(19) Europäisches Patentamt
European Patent Office
Office européen des brevets

(11) Publication number: **0 141 640**
**B1**

(12) # EUROPEAN PATENT SPECIFICATION

(45) Date of publication of patent specification: **22.07.87**

(51) Int. Cl.$^4$: **A 61 F 2/60,** A 61 F 2/70

(21) Application number: **84307463.4**

(22) Date of filing: **30.10.84**

(54) **Electrically released prosthetic joint.**

(30) Priority: **31.10.83 GB 8329018**

(43) Date of publication of application:
**15.05.85 Bulletin 85/20**

(45) Publication of the grant of the patent:
**22.07.87 Bulletin 87/30**

(84) Designated Contracting States:
**DE FR NL**

(56) References cited:
**GB-A-1 585 256**
**GB-A-2 099 708**
**US-A-4 074 367**

(73) Proprietor: **J.E. HANGER & COMPANY LIMITED**
**Roehampton Lane**
**Roehampton London SW15 5PL (GB)**

(72) Inventor: **Brown, David John**
**13 Luccombe Road Upper Shirley**
**Southampton Hampshire (GB)**

(74) Representative: **Cole, Paul Gilbert et al**
**Hughes Clark Byrne & Parker 63 Lincoln's Inn**
**Fields P.O. Box 22**
**London WC2A 3JU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 141 640 B1**

**Description**

The present invention relates to an electrically released prosthetic joint such as a knee joint for an artificial leg and particularly to a so-called primary artificial leg that has an electrically operated release mechanism.

We have previously described and claimed in Patent Specification GB—A—2099708 a knee joint for an artificial leg that is of mechanically simple construction but is nevertheless durable and effective. It comprises a knee member having a part spherical convex lower surface pivoted about an axis to a knee housing having a part spherical concave upper surface conforming to that of the knee member. The knee member has a springloaded locking plunger posterior to the pivot axis and having a generally vertical line of action that as the knee is straightened from a flexed position is retracted as it travels over a rib upstanding from the curved top surface of the knee housing, until the fully unflexed position is reached, when the locking plunger latches into a seating or socket in the knee housing to prevent flexion of the joint.

It is an object of the invention to provide an electrically operated release mechanism for the knee joint aforesaid that can be operated reliably with economy of power and without undue power drain in the event of a failure to release. Although particularly intended for a knee joint, the present invention is also applicable to other kinds of prosthetic joints that are releaseably locked by engagement of a spring loaded plunger into a socket.

The invention therefore provides a joint for a prosthesis having first and second members pivoted together for relative angular movement and a spring loaded plunger in one member that latches into a socket in the other member to hold the members in one angular position, wherein a solenoid is operatively connected to the plunger so that energisation thereof unlatches the plunger from the socket, a sensor detects unlatching movement of the solenoid and a circuit responsive to actuation of an operating switch and to the sensor is arranged to reduce the current through the solenoid to a holding value if unlatching movement occurs within a set time after actuation of the operating switch and in the absence of unlatching movement within that time substantially de-energises the solenoid.

An embodiment of the invention will now be illustrated in the accompanying drawings in which:

Figure 1 shows in vertical section the region adjoining the knee of an artificial leg showing schematically an associated solenoid and control unit; and

Figure 2 is a circuit diagram of the solenoid and its associated parts.

In Figure 1 a thigh stump socket (not shown) is fixed to a knee member in the form of a shaped block 10 of wood, polyurethane foam or other conveniently worked material. The height of block 10 may be adjusted e.g. by cutting with a saw to meet the needs of an individual patient for whom the limb is intended. A relatively large vertical bore 13 from the top face of block 10 leads to a smaller bore 14 that opens through the lower face of the knee block which is part spherical at a position posterior of the position of knee bolt and bearings 12. An extension buffer 33 mounted on a plate 33a is let into the curved lower face of the knee member 10 below bolt 12 as viewed in Figure 1. The portion of the block 10 posterior of the extension buffer 33 is formed with a channel 11 or is otherwise relieved, and a plunger 18 in housing 16 is mounted in the bore 14 so that it has a vertical line of action and in its position of maximum downward extension the tip projects beyond the relieved portion of the block surface but not significantly beyond the envelope of curvature of the lower face of the block. By this means, as will become apparent below, the tip of the plunger cannot latch onto the top anterior edge of the knee socket and thereby accidentally lock the knee in a fully flexed or hyperflexed state. Plunger 18 is urged downwardly by coil spring 17 in housing 16, has bearing portions towards its upper and lower ends that slideably guide it in the housing for vertical movement and is directly connected at its upper end to Bowden cable 25 which may be pulled upwardly to release the joint. Plunger 18 has a conical tip provided with an anti-friction pip 22 of Teflon or like material.

The knee housing 30 may be a casting in light alloy or an injection moulding in glass filled nylon and is pivoted to the knee member by bolt 12. Its upper face presents a part spherical concave surface conforming generally to the curvature of the lower face of the knee member. It is supported on a pylon tube 32 to which it is connected by a demountable compression joint. The upper face of the housing 30 is formed with an upstanding rib 29 whose curved top face forms a smooth track leading to a spigot 31 also upstanding from the top face of the housing 30 at a posterior location. The track defined by rib 29 leads to a frustoconical seating or socket 34 into which the tip of plunger 18 locates when the knee and shin portions of the leg are in the unflexed position shown. By this means the plunger and seating automatically take up wear so that the knee has no play in its unflexed locked state that would be damaging to patient confidence. The knee is locked both against flexion and against over-extension by the plunger 18. A solenoid 40 is secured in fixed position above the plunger 18 and has a moving element 41 connected to the cable 25 thereby directly to raise and disengage plunger 18 from socket 34 after which anti-friction pip 24 travels over the top face of rib 29 as the knee flexes, the said rib either supporting the retracted plunger and allowing it to extend gradually as the knee flexes.

The fully unflexed position of the knee is detected by a microswitch 42 in the knee member 10 that is tripped by the top anterior edge of knee housing 10. As the knee is returned from the flexed to the straight position the plunger 18 travels back over the top face of rib 29 by which it is retracted into the housing 16 and automatically latches back into the seating or socket 34. The extension buffer 33 and plate 33a are formed with a notch to accommodate the rib 29 as the knee is unflexed.

The solenoid 40 is powered from a battery 43 under the control of a control unit 44 and retraction of the plunger 18 is initiated by operation of an operating switch 45. The control unit 44 supplies current to a light-emitting diode 46 and responds to phototransistor 47 that together form an optical switch. It is arranged that the beam between diode 46 and transistor 47 is uninterrupted when the plunger 18 is home in the socket 34, but is interrupted when moving element 41 of the solenoid 40 has risen to unlatch the plunger 18, bringing blade on element 41 into the light path. Detection of unlatching is used to adjust the solenoid current as described more fully below and thereby prolong battery life. Also connected to control unit 44 is microswitch 42 that detects when the leg is unflexed. In a condition of slight leg flexion high lateral loads are imposed on plunger 18 and socket 34 thereby making it difficult to disengage the plunger 18 therefrom. Consequently control unit 44 is enabled to pass power to solenoid 40 only when the state of micro-switch 42 indicates that the leg is at its fully unflexed position.

Figure 2 shows those parts of the control circuit that pertain to the solenoid. The voltage V is applied across resistor $R_1$ of value typically 100 KΩ connected in series with capacitor $C_1$ and the junction therebetween is connected to input 2 of NAND gate 49, the input 1 being connected to the output of opto-switch 46, 47. The normal state of switch 42 is that it is connected to a 100Ω resistor $R_2$ across capacitor $C_1$ which is thereby discharged. On actuation of the switch 45 the capacitor $C_1$ is charged through $R_1$ and reaches a potential sufficient to influence the logic state of gate 49 after an appropriate time, typically one second. At power on the solenoid is down and the output from the optical switch is applied at input 1 logic 1 but the voltage from capacitor $C_1$ applied at input 2 is logic 0 and the output 3 is logic high and is fed through resistor $R_3$ to the base of transistor $Tr_1$ which then becomes conductive. Current flowing therethrough from the base of first power supply control transistor $Tr_2$ via resistor $R_4$ then causes $Tr_2$ which is normally non conductive by reason of high value biasing resistor $R_5$ to become conductive. Because the output of opto-switch 46, 47 is high, transistor $Tr_3$ and current flows via $R_6$ to the base of second power control resistor $Tr_4$ that is otherwise biased to a less conductive state by biasing resistor $R_7$. In consequence second power control transistor $Tr_4$ has maximum collector-emitter conductivity and series connected transistors $Tr_2$, $Tr_4$ pass about 2 amps to solenoid 40 which is sufficient to lift the plunger 18. If the plunger moves within the one second time constant of $R_1$, $C_1$, blade 48 interrupts the light beam, the output of optical switch 46, 47 falls and transistor $Tr_3$ becomes non-conductive. The base of transistor $Tr_4$ is now biased conductive by resistor $R_7$. The logic values at inputs 1, 2 of gate 49 are both low, so the output at 3 remains logic high and transistors $Tr_1$, $Tr_2$ remains conductive. Therefore transistors $Tr_2$, $Tr_4$ pass a holding current of about 300 mA to the solenoid 40 which is sufficient to maintain the plunger 18 retracted. If however the plunger 18 does not move within the time constant of $R_1$, $C_1$ the inputs 1, 2 to gate 49 both become logic 1 and the output at 3 becomes logic 0, thereby rendering transistors $Tr_1$ and $Tr_2$ non-conductive. The only current through solenoid 40 is then from transistor $Tr_3$ via the base of $Tr_4$ which is insufficient to bring about rapid discharge of battery 43. It will therefore be appreciated that effective operation of solenoid 40 is brought about with maximum battery power economy.

It will be appreciated that various modifications may be made to the embodiment described above without departing from the invention, the scope of which is defined in the appended claims.

**Claims**

1. A joint for a prosthesis having first and second members (10, 30) pivoted together for relative angular movement and a spring loaded plunger (18) is one member (10) that latches into a socket (34) in the other member (30) to hold the members (10, 30) in one angular position, characterised in that a solenoid (40) is operatively connected to the plunger (18) so that energisation thereof unlatches the plunger (18) from the socket (34), a sensor (46, 47) detects unlatching movement of the solenoid (40) and a circuit (44) responsive to actuation of an operating switch (45) and to the sensor (46, 47) is arranged to reduce the current through the solenoid (40) to a holding value if unlatching movement occurs within a set time after actuation of the operating switch (45) and in the absence of unlatching movement within that time substantially deenergises the solenoid (40).

2. A joint according to Claim 1 wherein the solenoid (40) is mounted at a fixed spacing from the plunger (18) and is connected to one end of a Bowden cable (25) whose other end is connected to the plunger (18).

3. A joint according to Claim 1 or 2, wherein the sensor is an optical source (46) and detector (47), light transmission therebetween being altered as a moving element (48) of the solenoid is displaced from the plunger latched position.

4. A joint according to Claim 3, wherein the optical detector output is fed to a first input of a logic gate (49) whose second input is controlled by a capacitor and resistor ($C_1$, $R_1$) that determines the set time and the output of the logic gate is fed to the base of a switching transistor ($Tr_1$) that controls the base of a first power supply control transistor ($Tr_2$) whose collector and emitter provide the current path to the solenoid (40), the said path being conductive when the output of the gate (49) is in one logic state only.

5. A joint according to claim 4 wherein the current path to the solenoid (40) is between collector and emitter of a second series connected power supply control transistor ($Tr_3$) whose base is switched between first and second bias states depending on the state of the optical switch (46, 47), so as to give the high solenoid current when the plunger (18) is latched and the lesser holding current when the solenoid (40) is unlatched.

6. A joint according to any preceding claim, further comprising a switch (42) responsive when said members (10, 30) are in said one angular position and said circuit (44) responds to the state of said switch (42) to enable power supply to said solenoid (40) only when said members (10, 30) are in said one angular position.

7. A joint according to any preceding claim which is a knee joint for an artificial leg comprising a knee member (10) having a part spherical convex lower surface pivoted about an axis (12), a knee housing (30) having a part spherical concave upper surface conforming to that of the knee member, the knee member (10) having the locking plunger (18) located posterior to the pivot axis and having a generally vertical line of action which as the joint is straightened from a flexed position travels over a rib (29) upstanding from the upper surface of the knee housing by which it is retracted and when the unflexed position has been reached is urged by a spring (17) into latching engagement with the socket (34) which is in the knee housing (30) to prevent flexion of the knee.

**Patentansprüche**

1. Gelenk für eine Prothese, die ein erstes und ein zweites Glied (10, 30) aufweist, die für eine relative Winkelbewegung drehbar aneinander befestigt sind, und einen feder-beaufschlagten Dorn (18) in einem Glied (10), der in einer Aufnahme (34), in dem anderen Glied (30) einklinkt, um die Glieder (10, 30) in einer Winkelposition zueinander zu halten, dadurch gekennzeichnet, daß eine Spule (40) wirkmäßig mit dem Dorn (18) derart verbunden ist, daß deren Erregung den Dorn (18) aus der Aufnahme (34) ausklinkt, daß ein Sensor (46, 47) die Ausklinkbewegung der Spule (40) erfaßt und daß ein Kreis (44) der auf die Betätigung eines Betätigungsschalters (45) und den Sensor (46, 47) entspricht, angeordnet ist, um den Strom durch die Spule (40) bis zu einem Haltewert zu reduzieren, falls die Entriegelungsbewegung innerhalb einer gesetzten Zeit nach der Aktion des Betätigungsschalters (45) auftritt, und in der Abwesenheit der Entriegelungsbewegung innerhalb dieser Zeit im wesentlichen die Spule (40) abregt.

2. Gelenk nach Anspruch 1, bei dem die Spule (40) in einem feststehenden Abstand von dem Dorn (18) befestigt ist und mit einem Ende eines Bowdenzuges (25) verbunden ist, dessen anderes Ende mit dem Dorn (18) verbunden ist.

3. Gelenk nach Anspruch 1 oder 2, bei dem der Sensor eine optische Quelle (46) und ein Detektor (47) ist, wobei die Lichtübertragung zwischen diesen beiden geändert wird, wenn ein sich bewegendes Element (48) der Spule von der Einrastposition des Dornes verschoben wird.

4. Gelenk nach Anspruch 3, bei dem der optische Detektor-Output einem ersten Input eines logischen Gitters (49) zugeführt wird, dessen zweiter Input von einer Kapazität und einem Widerstand ($C_1$, $R_1$) gesteuert wird, der die Setzzeit bestimmt, und wobei der Output des logischen Gitters der Basis eines Schalttransistors ($Tr_1$) zugeführt wird, welcher die Basis eines ersten Stromversorgungskontrolltransistors ($Tr_2$) kontrolliert, dessen Kollektor und Emitter einen Stromfluß zur Spule (40) hervorrufen, wobei dieser Weg dann leitend ist, wenn der Output des Gitters 49 in einem nur logischen Status ist.

5. Gelenk nach Anspruch 4, bei dem der Stromweg zu der Spule (40) zwischen dem Kollektor und dem Emitter einer zweiten Serie verbunden ist mit einem Stromversorgungskontrolltransistor ($Tr_3$), dessen Basis zwischen einem ersten und zweiten Spannungszustand abhängig von dem Zustand des optischen Schalters (46, 47) so geschaltet wird, daß ein hoher Spulenstrom abgegeben wird, wenn der Dorn (18) eingerastet ist, und ein weniger hoher Strom, wenn die Spule (40) ausgerastet ist.

6. Gelenk nach einem der vorangehenden Ansprüche, das darüber hinaus einen Schalter (42) aufweist, der dann anspricht, wenn die Teile (10, 30) in der genannten einen Winkelposition sind und der Kreis (44) auf den Status des Schalters (42) anspricht, um die Stromzufuhr zu der Spule (40) nur dann zu ermöglichen, wenn sich die Teile (10, 30) in der einen Winkelposition befinden.

7. Gelenk nach einem der vorangehenden Ansprüche, das ein Kniegelenk für ein künstliches Bein darstellt, mit einem Knieteil (10), das einen ballig-konvexen unteren Oberflächenbereich, um eine Achse (12) drehbar aufweist, ein Kniegehäuse (30), welches einen ballig-konkaven oberen Oberflächenbereich aufweist, der an das Knieteil angepaßt ist, wobei das Knieteil (10) einen Blockierdorn (18) aufweist, der hinter der Drehachse angeordnet ist und der eine im wesentlichen vertikale Wirklinie aufweist, der dann, wenn das Gelenk von einer gebeugten Position gestreckt wird, über eine Rippe (29), die von der oberen Oberfläche des Kniegehäuses absteht, wandert, von der er zurückgezogen wird, und der, wenn die ungebeugte Position erreicht wird, von einer Feder (17) in blockierenden Eingriff mit der Sitzfläche (34), die sich in dem Gehäuse (30) befindet, gezwungen wird, um die Biegung des Knies zu verhindern.

**Revendications**

1. Joint pourprothèse comportant un premier et second élément (10, 30) pouvant pivoter l'un par rapport à l'autre pour effectuer un mouvement angulaire relatif, et un plongeur poussé par un ressort (18) placé dans un élément (10) de manière à se verrouiller dans une alvéole (34) de l'autre élément (30) pour maintenir les éléments (10, 30) dans une position angulaire donnée, joint caractérisé en ce qu'un solénoïde (40) est relié en fonctionnement au plongeur (18) de façon que l'excitation de ce solénoïde déverrouille le plongeur (18) de l'alvéole (34), en ce qu'un détecteur (46, 47) détecte le mouvement de déverrouillage du solénoïde (40), et en ce qu'un circuit (44) répondant au fonctionnement d'un commutateur de manoeuvre (45) et du détecteur (46, 47), est utilisé pour réduire à une valeur de maintien le courant traversant le solénoïde (40) si le mouvement de déverrouillage se produit pendant une période de temps préréglée après le fonctionnement du commutateur de manoeuvre (45), et pour couper pratiquement l'excitation du solénoïde (40) en l'absence de mouvement de déverrouillage pendant cette période de temps.

2. Joint selon la revendication 1, caractérisé en ce que le solénoïde (40) est monté à une distance fixe du plongeur (18), et se relie à une extrémité d'un câble Bowden (35) dont l'autre extrémité est reliée au plongeur (18).

3. Joint selon l'une quelconque des revendication 1 et 2, caractérisé en ce que le détecteur est constitué par une source optique (46) et un détecteur optique (47), la transmission de lumière entre les deux étant modifiée, lorsqu'un élément mobile (48) du solénoïde est déplacé à partir de la position de verrouillage du plongeur.

4. Joint selon la revendication 3, caractérisé en ce que le signal de sortie du détecteur optique est appliqué à une première entrée d'une porte logique (49) dont la seconde entrée est commandée par un condensateur et un résistance ($C_1$, $R_1$) déterminant le temps de réglage, et en ce que le signal de sortie de la porte logique est appliqué à la base d'un transistor de commutation ($Tr_1$) commandant la base d'un premier transistor de commande d'alimentation de puissance ($Tr_2$) dont le collecteur et l'émetteur forment le chemin de passage du courant alimentant le solénoïde (40), ce chemin de passage n'étant conducteur que lorsque la sortie de la porte (49) se trouve dans un certain état logique.

5. Joint selon la revendication 4, caractérisé en ce que le chemin de passage du courant alimentant le solénoïde (40) est formé entre le collecteur et l'émetteur d'un second transistor de commande d'alimentation de puissance branché en série ($Tr_3$) dont la base est commutée entre un premier et second état de polarisation dépendant de l'état de commutateur optique (46, 47), de manière à fournir le courant d'alimentation élevé du solénoïde, lorsque le plongeur (18) est verrouillé, et à fournir le courant de maintien plus faible lorsque le solénoïde (40) est déverrouillé.

6. Joint selon l'une quelconque des revendications précédentes, caractérisé en ce qu'il comprend en outre un commutateur (42) répondant au fait que les éléments (10, 30) se trouvent dans la position angulaire donnée, et en ce que le circuit (44) répond à l'état du commutateur (42) pour ne laisser passer la puissance d'alimentation du solénoïde (40) que lorsque les éléments (10, 30) se trouvent dans cette position angulaire donnée.

7. Joint selon l'une quelconque des revendications précédentes, caractérisé en ce qu'il consiste en un joint de genou pour jambe artificielle comprenant un élément de genou (10) comportant une surface inférieure convexe partiellement sphérique pouvant pivoter autour d'un axe (12), un logement de genou (30) comportant une surface supérieure concave partiellement sphérique se conformant à la surface de l'élément de genou, le plongeur de verrouillage (18) de cet élément de genou (10) étant placé postérieurement à l'axe de pivot de comportant une ligne d'action généralement verticale qui, lorsque le joint est redressé à partir d'une position fléchie, passe sur une nervure (29) faisant saillie verticalement sur la surface supérieure du logement de genou permettant de le rétracter, et qui, lorsque la position déployée a été atteinte, se trouve poussé par un ressort (7) pour venir se verrouiller dans l'alvéole (34) se trouvant dans le logement de genou (30), de manière à empêcher la flexion du genou.

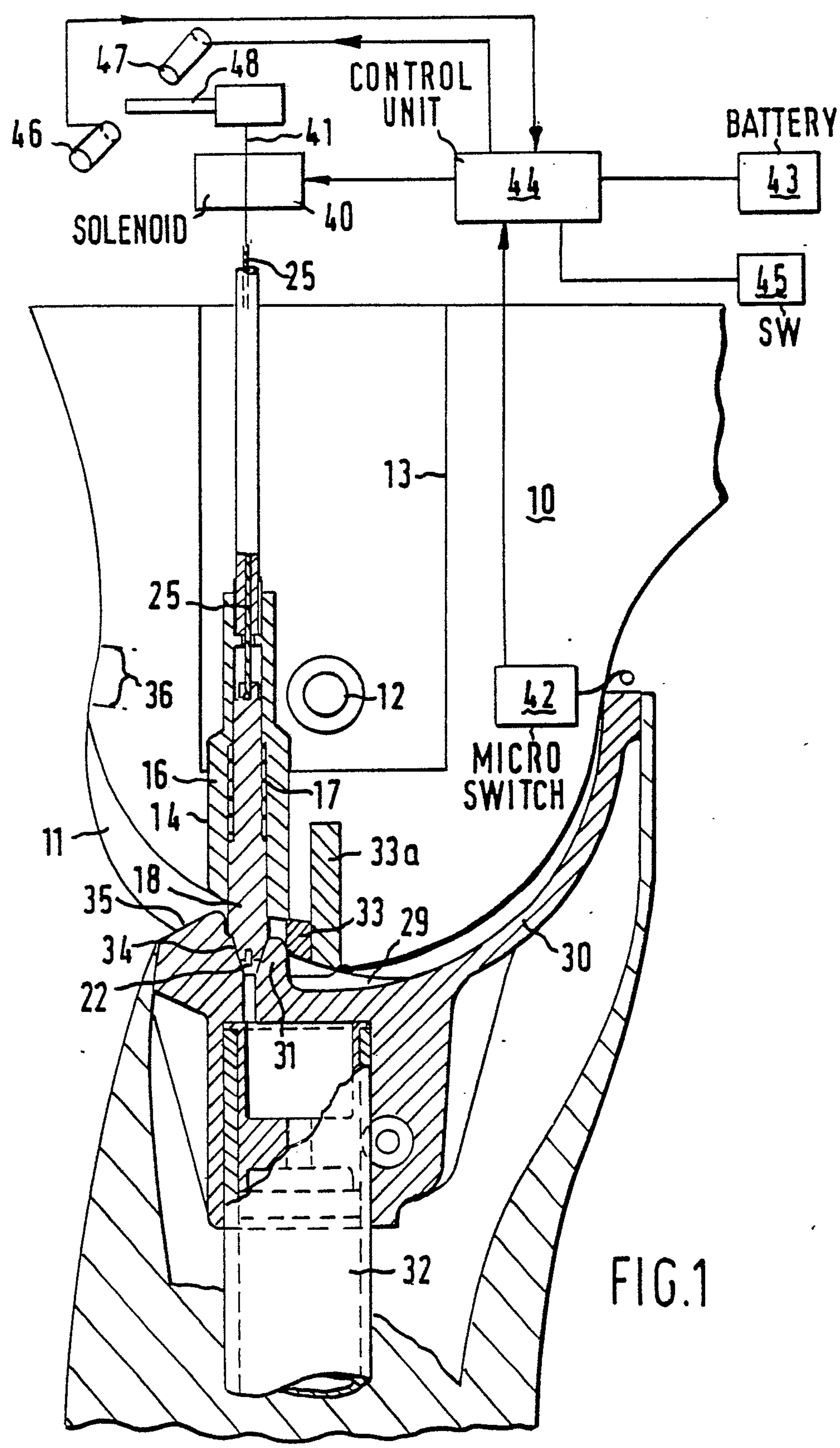
47
48
46
CONTROL UNIT
41
BATTERY
44
43
SOLENOID
40
25
45
SW
13
10
25
36
12
42
MICRO SWITCH
16
17
14
11
33a
18
35
33
29
34
30
22
31
32

FIG.1

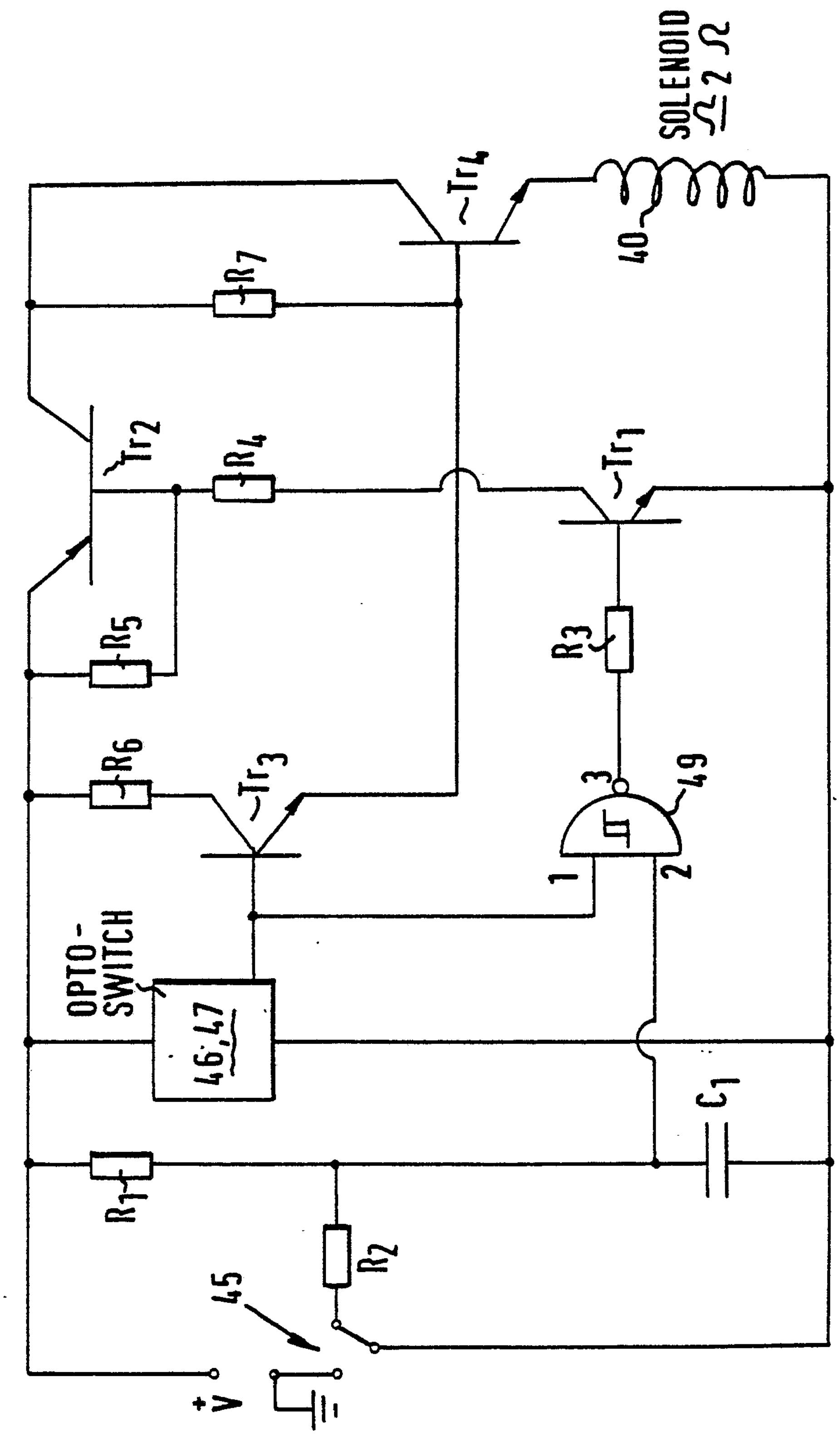
SOLENOID
2 Ω
40
Tr4
R7
Tr2
R4
Tr1
R5
R3
R6
Tr3
3
49
1
2
OPTO-
SWITCH
46,47
C1
R1
R2
45
+V

FIG.2